(19) Europäisches Patentamt / European Patent Office / Office européen des brevets

(11) **EP 1 767 521 B1**

(12) **EUROPEAN PATENT SPECIFICATION**

(45) Date of publication and mention
of the grant of the patent:
**23.04.2014 Bulletin 2014/17**

(21) Application number: **05765086.3**

(22) Date of filing: **24.06.2005**

(51) Int Cl.:
*C07C 229/26* [(2006.01)]     *C07C 51/41* [(2006.01)]
*C07C 51/43* [(2006.01)]     *C07C 59/265* [(2006.01)]
*C07C 227/16* [(2006.01)]     *C07C 227/42* [(2006.01)]
*C07C 229/36* [(2006.01)]

(86) International application number:
**PCT/JP2005/011635**

(87) International publication number:
**WO 2006/001378 (05.01.2006 Gazette 2006/01)**

(54) **L-ORNITHINE-CITRIC ACID SALT CRYSTAL**

L-ORNITHIN-CITRONENSÄURESALZKRISTALL

CRISTAL DE L-ORNITHINE CITRATE

(84) Designated Contracting States:
**AT BE BG CH CY CZ DE DK EE ES FI FR GB GR
HU IE IS IT LI LT LU MC NL PL PT RO SE SI SK TR**

(30) Priority: **25.06.2004 JP 2004188813**

(43) Date of publication of application:
**28.03.2007 Bulletin 2007/13**

(73) Proprietor: **Kyowa Hakko Bio Co., Ltd.
Tokyo, 100-8185 (JP)**

(72) Inventors:
• **MURATA, Hideki c/o Technical Research
Laboratories
Yamaguchi 747- 8522 (JP)**

• **YAMAMOTO, Yu
Fukuoka 811-1353 (JP)**

(74) Representative: **Casalonga, Axel
Casalonga & Partners
Bayerstrasse 73
80335 München (DE)**

(56) References cited:
**JP-A- 4 229 184     JP-A- 55 136 254
JP-A- 2000 086 482     JP-A- 2003 144 088**

**Description**

Technical Field

The present application relates to a crystal of a salt of L-ornithine and citric acid (L-ornithine-citric acid salt) and a process for producing the same.

Background Art

L-ornithine has been widely used as an ingredient of nutrition enriching additives, pharmaceuticals or the like.
Since it is difficult to prepare a free base of L-ornithine as a crystal, it is available usually in the form of a salt such as hydrochloride (Product Catalogue 2004 to 2005 of Sigma) .
When L-ornithine is used as an ingredient of a transfusion or the like for the purpose of nutrition enriching or the like, for example, by using its hydrochloride is used as it is, an acidosis symptom may be induced. Also, administration of a transfusion containing a large amount of chlorine ions is unfavorable for patients with a renal disease in particular. It has also been well known that when L-ornithine is used either by being mixed in foods or the like as a nutrition enriching additive or the like or orally as it is, it is difficult to utilize the same in the form of, for example, hydrochloride because of its bitter taste.
As a salt of L-ornithine, for example, $\alpha$-ketoglutarate (refer to Patent Document 1), L-aspartate (refer to Patent Document 2), malate (refer to Patent Document 3) and the like other than the hydrochloride described above are known as crystals.
Meanwhile, it is known that a salt of ornithine and citric acid can be used as a taste improver, but crystals thereof are not known yet (refer to Patent Document 4).

Patent Document 1: gazette of Japanese Published Examined Patent Application No. 3194/1971
Patent Document 2: gazette of Japanese Published Unexamined Patent Application No. 364155/1992
Patent Document 3: gazette of Japanese Published Unexamined Patent Application No. 136254/1980
Patent Document 4: gazette of Japanese Published Unexamined Patent Application No. 144088/2003

Disclosure of the application

Problems to be Solved by the application

An object of the present application is to provide a crystal of an L-ornithine-citric acid salt excellent as a supply source of L-ornithine and a process for producing the same.

Means for Solving the Problems

The application relates to the following (1) to (5).

(1). A crystal of a salt of L-ornithine and citric acid, according to claim 1.
(2). A process for producing a crystal of an L-ornithine-1/2 citric acid salt, which comprises adding citric acid to an aqueous solution containing a free base of L-ornithine, wherein citric acid is added in an amount of 0.4 to 0.6 equivalent to L-ornithine and the pH of the aqueous solution is adjusted to a value of 4 to 5 by adding citric acid, and precipitating the crystal from the resulting aqueous solution by adding an hydrophilic organic solvent to the resulting aqueous solution.
(3). The process according to claim 2, wherein the hydrophilic organic solvent is an alchols, an amides, acetone or acatonitrile.
(4). The process according to claim 2, wherein the hydrophilic organic solvent is a solvent selected from the group conisisting of methanol, ethanol, propanol, isopropyl alcohol, butanol, ethylene glycol, diethylene glycol, N,N-dimethylformamide, N,N-dimethylacetamide, N-methylpyrrolidone, acetone and acetonitrile.
(5). The process according to claim 2, wherein the hydrophilic organic solvent is methanol or ethanol.

Effect

The present application provides a crystal of an L-ornithine-citric acid salt excellent as a supply source of L-ornithine and a process for producing the same.

Brief Description of the Drawing

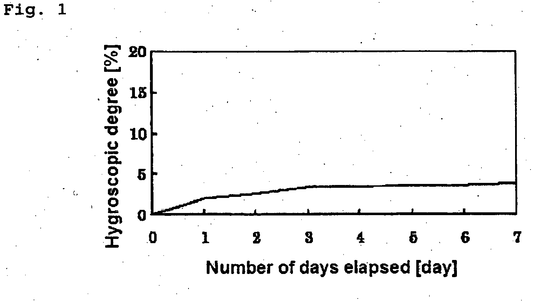

[Fig. 1] Fig. 1 shows test results of hygroscopicity of the crystals of an L-ornithine-1/2 citric acid salt obtained in Example 1. In the drawing, the abscissa represents the number of days elapsed (day) after starting the test, and the ordinate represents a hygroscopic rate (%).

Best Mode for Carrying Out the Invention

The crystal of the L-ornithine-citric acid salt in the present invention comprises preferably 0.5 to 3 mols of L-ornithine and 1 mol of citric acid, more preferably 2 mols of L-ornithine and 1 mol of citric acid. The aqueous solution thereof is neutral or acidic solution. In the 5 weight % aqueous solution thereof, the pH of the solution is 3 to 7, preferably 3 to 6, more preferably 4 to 5, most preferably 4.6 to 5.0.

While the crystal usually exists singly, it may exist as a solvate with water or various organic solvents, and these solvates are also comprised in the present invention.

Examples of the hydrophilic organic solvent used in the invention include alchols such as methanol, ethanol, propanol, isopropyl alcohol, butanol, ethylene glycol or diethylene glycol; amides such as N,N-dimethylformamide, N,N-dimethylacetamide or N-methylpyrrolidone; acetonitrile; acetone or the like. Methanol or ethanol is preferable. These solvents can be used alone or in combination thereof.

The process for producing the crystals of the L-ornithine ½ citric acid salt in the present application is described below.

For example, a culture solution, a concentrated culture solution or the like containing a salt of L-ornithine such as L-ornithine hydrochloride or L-ornithine which is commercially available is treated with a strongly acidic ion exchange resin and the like, to obtain an aqueous solution containing a free base of L-ornithine. Citric acid is added to the resulting aqueous solution of a free base of L-ornithine, and dissolved therein. To obtain the crystal of the present invention in which an L-ornithine to citric acid composition ratio is 2:1 (molar ratio), citric acid is added in an amount of 0.4 to 0.6 equivalent, most preferably 0.5 equivalent to L-ornithine, and the resulting solution is adjusted to pH of, 4 to 5, most preferably 4.6 to 5.0.

In order to efficiently obtain the crystal of the L-ornithine-citric acid salt from the resulting aqueous solution, the crystals are isolated by precipitation thereof by addition of the hydrophilic organic solvent to the aqueous solution. When adding the hydrophilic organic solvent to the aqueous solution or the crystals may partially be precipitated in the aqueous solution.

When adding the hydrophilic organic solvent to the aqueous solution to precipitate the crystals, the aqueous solution is preferably used by conducting a procedure such as evaporation for adjusting the concentration of the L-ornithine-citric acid salt to 30 to 80%, preferably 40 to 60%. When the aqueous solution having the concentration of 30% ormore is used, the amount of the hydrophilic organic solvent used to precipitate the crystals can be decreased, which is more preferably. When the aqueous solution having the concentration of 80% or less is used, the aqueous solution and the hydrophilic organic solvent are mixed more easily, and the precipitation of the crystals is induced easily. In addition, conversion of the precipitated crystals to a blocked aggregate is easily avoidable. The hydrophilic organic solvent is used in an amount which is usually 1 to 8 times (water content is approximately 50% to 11%), preferably 1.5 to 5 times (water content is approximately 40 to 17%), more preferably 2 to 3 times (water content is approximately 33 to 25%) as large as the amount of the above aqueous solution which is adjusted the concentration. And the solvent is added gradually or dropwise at -10°C to 60°C, preferably -10°C to room temperature to the above aqueous solution which is adjusted the concentration. The crystals are sometimes precipitated by mere addition of the hydrophilic organic solvent, but, the crystals are usually precipitated by stirring the resulting mixed solution at -10°C to room temperature for 5 minutes to 72 hours. The crystals separately obtained by the present invention can be used as seed crystals and added to the resulting mixed solution to induce the precipitation of the crystals. Seed crystals are sufficient so long as crystallization can be induced, and used in an amount of 0.01 to 0.1%, preferably 0.05% to the amount of the L-ornithine-citric acid salt contained in the mixed solution.

When adding the aqueous solution to the hydrophilic organic solvent to precipitate the crystals, the crystals can be precipitated by dropping the aqueous solution which is adjusted the concentration to the hydrophilic organic solvent at -10°C to 60°C, preferably -10°C to room temperature with stirring described above. The hydrophilic organic solvent is usually used in an amount which is 1 to 8 times as large as the amount of the above aqueous solution which is adjusted the concentration.

The crystals of the L-ornithine-citric acid salt can be obtained by separating the precipitated crystals via filtration or the like and drying the same.

Crystals of a salt of another basic amino acid (for example, lysine, arginine and the like) and citric acid may be obtained in the same manner.

Next, the hygroscopicity of the crystals of the L-ornithine-citric acid salt in the present invention is described below in Test Example.

Test Example 1

A saturated aqueous solution of sodium chloride (approximately 200 mL) was allowed to stand in a plastic desiccator at 25°C for 24 hours (relative humidity was adjusted to 75%) . The crystals (2 g) prepared in Example 1 were weighed out in a glass weighing vessel. The weighing vessel was allowed to stand in the desiccator with the humidity adjusted, and the weight change of the weighing vessel was measured over the course of time.

The hygroscopicity (hygroscopic degree) was calculated using the following formula (1) on the basis of the weight change at each measuring time. The measurement of the weight change of the weighing vessel was continued until day 7.

[Formula 1]

$$\text{Hygroscopic rate (\%)} = \text{W3-W2/W2-W1} \times 100 \quad (1)$$

W1 = Weight (g) of a weighing vessel
W2 = Weight (g) of a weighing vessel filled with crystals before the test
W3 = Weight (g) of a weighing vessel filled with crystals after the test

The results are shown in Fig. 1.

The crystals of the L-ornithine-citric acid salt prepared in Example 1 did not show notable hygroscopicity. Therefore, it was shown that there was no need to pay special attention to humidity in storage of the L-ornithine-citric acid salt of the present invention.

Also, the L-ornithine-citric acid salt prepared in Example 3 did not have a bitter taste.

As described above, the crystals of the L-ornithine-citric acid salt in the present invention can be stored in an ambient atmosphere at room temperature. The crystals of the L-ornithine-citric acid salt are excellent as a supply source of L-ornithine because during the process for producing the same, corrosive chemicals such as hydrochloric acid are not used and citric acid is less costly in comparison with L-malic acid and the like.

Moreover, the crystals of the L-ornithine-citric acid salt are expected to have properties provided by citric acid in addition to the effect inherent in L-ornithine. Citric acid is an intermediate of a citric acid cycle (TCA cycle). Through activation thereof, an effect of recovery from fatigue can be expected by acceleration of fatty acid synthesis and elimination of lactic acid accumulated in the body. Further, improvement of a bitter taste provided by hydrochloride or the like is also expected. Therefore, the crystals of the L-ornithine-citric acid salt are expected to be used as a better supply source of L-ornithine in nutrition enriching additives or pharmaceuticals.

The present invention is illustrated specifically below by referring to Examples. However, the invention is not limited to these Examples.

Example 1

L-ornithine hydrochloride (20 g, 15.7 g as free L-ornithine) was dissolved in water (400 mL), and the solution was passed through a column filled with a strongly acidic ion exchange resin (Marason C, H-type, 200 mL). After the resin was washed with water (200 mL), L-ornithine was eluted with 2 mol/L aqueous ammonia (400 mL). The eluate was concentrated to approximately 200 mL under reduced pressure, and citric acid (12 g, 0.5 equivalent to L-ornithine) was then added to the resulting aqueous solution of L-ornithine (containing 0.119 mol of L-ornithine) to adjust the pH of the aqueous solution to 4.7. The resulting aqueous solution was concentrated under reduced pressure to adjust the total volume to 45 mL. Subsequently, ethanol (105 mL) was gradually added to the solution with stirring at room temperature. After the mixture was further stirred at room temperature for 20 hours, the precipitated crystals were collected by filtration, and washed with ethanol. The resulting crystals were dried overnight at 20°C under reduced pressure to give crystals of L-ornithine-1/2 citric acid salt (25.1 g, yield: 92.7%) as pale yellow massive crystals.

Melting point: 165.5°C

IR spectrum (KBr, cn$^{-1}$): 1288.4, 1033.8, 948.9, 803.3, 715.5.

X-ray powder diffraction analysis: it was measured by RAD-X type (manufactured by Rigaku Denki). The results are shown in Table 1.

[Table 1]

Table 1

| Analytical angle (2θ (theta)) | Peak intensity (Relative intensity) |
|---|---|
| 6.05 | 21 |
| 11.50 | 62 |
| 12.15 | 32 |
| 18.65 | 78 |
| 20.70 | 100 |
| 23.45 | 44 |
| 24.55 | 39 |
| 25.30 | 45 |
| 29.65 | 34 |
| 30.35 | 34 |

Analysis of crystal composition: the results are shown in Table 2.
[Table 2]

Table 2

| | Found (%)* | Calculated (%)** |
|---|---|---|
| L-ornithine(%) | 56.5 | 57.9 |
| Citric acid(%) | 43.5 | 42.1 |

(Notes) * : Each found value was calculated by analyzing L-ornithine via an OPA coloring method (excitation wavelength: 340 nm, fluorescence wavelength: 455 nm) and citric acid via a UV detection method (detection wavelength: 210 nm) using high-performance liquid chromatography (HPLC).
**: Each calculated value was calculated as $(C_5H_{12}N_2O_2)_2 \cdot C_6H_5O_7$.

Example 2

To an aqueous solution of L-ornithine (containing 0.119 mol of L-ornithine) prepared in the same manner as in Example 1 was added citric acid (12 g, 0.5 equivalent to L-ornithine) to adjust pH of the aqueous solution to 4.7. The resulting solution was concentrated under reduced pressure to adjust the total volume to 45 mL. Subsequently, methanol (105 mL) was gradually added to the solution with stirring at room temperature. After the mixture was further stirred at room temperature for 18 hours, the precipitated crystals were collected by filtration, and washed with methanol. The resulting crystals were dried overnight at 20°C under reduced pressure to give crystals of an L-ornithine-1/2 citric acid salt (24.8 g, yield: 91.6%) as pale yellow flaky crystals.
Melting point: 161.8°C
IR spectrum (KBr, cn$^{-1}$): 1287.4, 1044.4, 935.4, 808.1, 754.1.
X-ray powder diffraction: it was measured by RAD-X type (manufactured by Rigaku Denki). The results are shown in Table 3.
[Table 3]

Table 3

| Analytical angle (2θ (theta)) | Peak intensity (Relative intensity) |
|---|---|
| 6.50 | 33 |
| 7.00 | 21 |
| 13.05 | 22 |
| 14.05 | 100 |
| 15.50 | 25 |
| 18.50 | 46 |

(continued)

| Analytical angle (2θ (theta)) | Peak intensity (Relative intensity) |
|---|---|
| 19.35 | 94 |
| 20.70 | 33 |
| 23.75 | 28 |
| 24.60 | 59 |
| 28.40 | 39 |

Analysis of crystal composition: the results are shown in Table 4.
[Table 4]

Table 4

|  | Found (%)* | Calculated (%)** |
|---|---|---|
| L-ornithine(%) | 57.0 | 57.9 |
| Citric acid(%) | 43.0 | 42.1 |

(Notes) * : Each found value was calculated by analyzing L-ornithine via an OPA coloring method (excitation wavelength: 340 cm, fluorescence wavelength: 455 nm) and citric acid via a UV detection method (detection wavelength: 210 nm) using high-performance liquid chromatography (HPLC).
**: Each calculated value was calculated as $(C_5H_{12}N_2O_2)_2 \cdot C_6H_8O_7$.

Example 3

To an aqueous solution of L-ornithine (containing 0.119 mol of L-ornithine) obtained in the same manner as in Example 1 was added citric acid (12 g, 0.5 equivalent to L-ornithine) to adjust pH of the aqueous solution to 4.7. The resulting solution was concentrated under reduced pressure to adjust the total volume to 68 mL. Subsequently, ethanol (272 mL) was gradually added to the solution with stirring at room temperature. After the mixture was further stirred at room temperature for 2 4 hours, the precipitated crystals were collected by filtration, and washed with ethanol. The resulting crystals were dried overnight at 20°C under reduced pressure to give crystals of L-ornithine-1/2 citric acid salt (25.5 g, yield: 94.0%) as pale yellow flaky crystals.
Melting point: 165.9°C
Analysis of crystal composition: the results are shown in Table 5.
[Table 5]

Table 5

|  | Found (%)* | Calculated (%)** |
|---|---|---|
| L-ornithine(%) | 57.2 | 57.9 |
| Citric acid(%) | 42.8 | 42.1 |

(Notes) * : Each found value was calculated by analyzing L-ornithine via an OPA coloring method (excitation wavelength: 340 nm, fluorescence wavelength: 455 nm) and citric acid via a UV detection method (detection wavelength: 210 nm) using high-performance liquid chromatography (HPLC).
** : Each calculated value was calculated as $(C_5H_{12}N_2O_2)_2 - C_6H_8O_7$.

Industrial Applicability

The crystal of the L-ornithine-citric acid salt, the process for producing the same and the like provided by the present application are useful as a supply source of L-ornithine

## Claims

1. A crystal of a salt of L-ornithine and citric acid, wherein:

    1) IR spectrum (KBr, cn$^{-1}$) is as follows: 1288.4, 1033.8, 948.9, 803.3, 715.5; and X-ray powder diffraction analysis is as follows:

| Analytical angle (2θ (theta)) | Peak intensity (Relative intensity) |
|---|---|
| 6.05 | 21 |
| 11.50 | 62 |
| 12.15 | 32 |
| 18.65 | 78 |
| 20.70 | 100 |
| 23.45 | 44 |
| 24.55 | 39 |
| 25.30 | 45 |
| 29.65 | 34 |
| 30.35 | 34 |

    or
    2) IR spectrum (KBr, cn$^{-1}$) is as follows: 1287.4, 1044.4, 935.4, 808.1, 754.1; and X-ray powder diffraction is as follows:

| Analytical angle (2θ (theta)) | Peak intensity (Relative intensity) |
|---|---|
| 6.50 | 33 |
| 7.00 | 21 |
| 13.05 | 22 |
| 14.05 | 100 |
| 15.50 | 25 |
| 18.50 | 46 |
| 19.35 | 94 |
| 20.70 | 33 |
| 23.75 | 28 |
| 24.60 | 59 |
| 28.40 | 39 |

2. A process for producing a crystal of an L-ornithine-1/2 citric acid salt, which comprises adding citric acid to an aqueous solution containing a free base of L-ornithine, wherein citric acid is added in an amount of 0.4 to 0.6 equivalent to L-ornithine and the pH of the aqueous solution is adjusted to a value of 4 to 5 by adding citric acid, and precipitating the crystal from the resulting aqueous solution by adding an hydrophilic organic solvent to the resulting aqueous solution.

3. The process according to claim 2, wherein the hydrophilic organic solvent is an alchols, an amides, acetone or acetonitrile.

4. The process according to claim 2, wherein the hydrophilic organic solvent is a solvent selected from the group consisting of methanol, ethanol, propanol, isopropyl alcohol, butanol, ethylene glycol, diethylene glycol. N,N-dimethylformamide, N,N-dimethylacetamide, N-methylpyrrolidone, acetone and acetonitrile.

**5.** The process according to claim 2, wherein the hydrophilic organic solvent is methanol or ethanol.


**Patentansprüche**

**1.** Kristall eines Salzes von L-Ornithin und Zitronensäure, wobei:

1) das IR-Spektrum (KBr, cn$^{-1}$) wie folgt aussieht: 1288.4, 1033.8, 948.9, 803.3, 715.5; und die Röntgenpulverbeugungsanalyse wie folgt lautet:

| Analytischer Winkel (2θ (Theta)) | Peakintensität (relative Intensität) |
|---|---|
| 6,05 | 21 |
| 11,50 | 62 |
| 12,15 | 32 |
| 18,65 | 78 |
| 20,70 | 100 |
| 23,45 | 44 |
| 24,55 | 39 |
| 25,30 | 45 |
| 29,65 | 34 |
| 30,35 | 34 |

oder

2) das IR-Spektrum (KBr, cn$^{-1}$) wie folgt aussieht: 1287.4, 1044.4, 935.4, 808.1, 745.1; und die Röntgenpulverbeugungsanalyse wie folgt lautet:

| Analytischer Winkel (2θ (Theta)) | Peakintensität (relative Intensität) |
|---|---|
| 6,50 | 33 |
| 7,00 | 21 |
| 13,05 | 22 |
| 14,05 | 100 |
| 15,50 | 25 |
| 18,50 | 46 |
| 19,35 | 94 |
| 20,70 | 33 |
| 23,75 | 28 |
| 24,60 | 59 |
| 28,40 | 39 |

**2.** Verfahren zur Herstellung eines Kristalls eines L-Ornithin-1/2-Zitronensäuresalzes, bei dem Zitronensäure einer wässrigen Lösung zugesetzt wird, die eine freie Base von L-Ornithin enthält, wobei die Zitronensäure dem L-Ornithin in einer Menge von 0,4 bis 0,6 Äquivalenten zugesetzt wird und der pH-Wert der wässrigen Lösung durch Zugabe von Zitronensäure auf einen Wert von 4 bis 5 eingestellt wird, und bei dem der Kristall durch Zugabe eines hydrophilen organischen Lösungsmittels zu der resultierenden wässrigen Lösung aus der resultierenden wässrigen Lösung ausgefällt wird.

**3.** Verfahren nach Anspruch 2, wobei es sich bei dem hydrophilen organischen Lösungsmittel um einen Alkohol, Amide, Aceton oder Acetonitril handelt.

**4.** Verfahren nach Anspruch 2, wobei das hydrophile organische Lösungsmittel ein Lösungsmittel ist, das aus der aus Methanol, Ethanol, Propanol, Isopropylalkohol, Butanol, Ethylenglycol, Diethylenglycol, N,N-Dimethylformamid, N,N-Dimethylacetamid, N-Methylpyrrolidon, Aceton und Acetonitril bestehenden Gruppe ausgewählt ist.

**5.** Verfahren nach Anspruch 2, wobei das hydrophile organische Lösungsmittel Methanol oder Ethanol ist.

**Revendications**

**1.** Cristal d'un sel de L-ornithine et d'acide citrique, duquel :

1) le spectre IR (KBr, cm$^{-1}$) est le suivant :

1288,4 1033,8 948,9 803,3 715,5 et le diagramme d'analyse de poudre par diffraction des rayons X est le suivant :

| Angle d'analyse 2 θ (thèta) | Intensité du pic (intensité relative |
|---|---|
| 6,05 | 21 |
| 11,50 | 62 |
| 12,15 | 32 |
| 18,65 | 78 |
| 20,70 | 100 |
| 23,45 | 44 |
| 24,55 | 39 |
| 25,30 | 45 |
| 29,65 | 34 |
| 30,35 | 34 |

2) ou le spectre IR (KBr, cm$^{-1}$) est le suivant :

1287,4 1044,4 935,4 808,1 754,1 et le diagramme d'analyse de poudre par diffraction des rayons X est le suivant :

| Angle d'analyse 2 θ (thèta) | Intensité du pic (intensité relative |
|---|---|
| 6,50 | 33 |
| 7,00 | 21 |
| 13,05 | 22 |
| 14,05 | 100 |
| 15,50 | 25 |
| 18,50 | 46 |
| 19,35 | 94 |
| 20,70 | 33 |
| 23,75 | 28 |
| 24,60 | 59 |
| 28,40 | 39 |

**2.** Procédé de production d'un cristal d'un sel hémi-citrate de L-ornithine qui comporte le fait d'ajouter de l'acide citrique à une solution aqueuse contenant de la L-ornithine à l'état de base libre, étant entendu que l'on ajoute l'acide citrique en une quantité de 0,4 à 0,6 équivalent, par rapport à la L-ornithine, et qu'en ajoutant cet acide citrique, on ajuste le pH de la solution aqueuse à une valeur de 4 à 5, et le fait de faire précipiter le cristal dans la solution aqueuse ainsi obtenue, en ajoutant à cette solution aqueuse résultante un solvant organique hydrophile.

**3.** Procédé conforme à la revendication 2, dans lequel le solvant organique hydrophile est un alcool, un amide, de l'acétone ou de l'acétonitrile.

**4.** Procédé conforme à la revendication 2, dans lequel le solvant organique hydrophile est un solvant choisi dans l'ensemble constitué par les suivants : méthanol, éthanol, propanol, alcool isopropylique, butanol, éthylèneglycol, diéthylèneglycol, N,N-diméthyl-formamide, N,N-diméthyl-acétamide, N-méthyl-pyrrolidone, acétone et acétonitrile.

**5.** Procédé conforme à la revendication 2, dans lequel le solvant organique hydrophile est du méthanol ou de l'éthanol.

Fig. 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 46003194 A
- JP 4364155 A
- JP 55136254 A
- JP 2003144088 A

**Non-patent literature cited in the description**

- *Product Catalogue,* 2004